# EUROPÄISCHE PATENTSCHRIFT

(11) **EP 0 508 133 B1**
(45) Veröffentlichungstag und Bekanntmachung des Hinweises auf die Patenterteilung: **15.02.1995**
(21) Anmeldenummer: 92104160.4
(22) Anmeldetag: 11.03.1992
(51) Int. Cl.: C07C 227/34, C07C 229/12

(54) **Verfahren zur Herstellung von L-Carnitin aus D,L-Carnitinnitrilsalzen**
Method of preparation of L-Carnitine from D,L-Carnitine nitrile salts
Procédé pour la préparation de L-Carnitine de sels du nitrile du D,L-Carnitine

(30) Priorität: 12.04.1991 DE 4111913
(43) Veröffentlichungstag der Anmeldung: 14.10.1992
(73) Patentinhaber: DEGUSSA AG, 60311 Frankfurt (DE)
(72) Erfinder: Jakob, Harald, Dr., W-6467 Hasselroth 1 (DE); Huthmacher, Klaus, Dr., W-6460 Gelnhausen (DE); Klenk, Herbert, Dr., W-6450 Hanau 9 (DE)

(56) Entgegenhaltungen:
- FR-A- 1 513 328
- FR-A- 2 536 391
- CHEMICAL ABSTRACTS, vol. 112, no. 9, 26. Februar 1990, Seite 763, Zusammenfassung Nr. 76804s, Columbus, Ohio, US; A. HORINAKA: "Resolution of dl-carnitinenitrile chloride as an intermediate for l-carnitine"; & JP-A-01 131 143
- PATENT ABSTRACTS OF JAPAN, vol. 12, no. 181 (C-499), 1987; & JP-A-62 286 959

## Beschreibung

Die Erfindung betrifft ein Verfahren zur Herstellung von L-Carnitin durch Racematspaltung von D,L-Carnitinnitrilsalzen unter Verwendung von optisch aktivem N-Acetyl-prolin als Spaltreagens und Verseifung des erhaltenen L-Carnitinnitrilsalzes.

L-Carnitin, auch als Vitamin B_{T} bekannt, findet in diätischen und pharmazeutischen Präparaten zunehmend Anwendung zur Behandlung von Herzmuskelschäden, chronischen Durchblutungsstörungen sowie zur Erhöhung der Leistungsfähigkeit. Die meisten chemischen Verfahren zur Herstellung von L-Carnitin schließen eine Racematspaltung einer Carnitin-Vorstufe ein. Bekannt ist eine Racematspaltung der Vorstufe D,L-Carnitinamidchlorid unter Verwendung einer optisch aktiven Säure (DD-PS 23217, DE-OS 29 27 672, DE-OS 33 42 713). Nachteilig ist insbesonders, daß D,L-Carnitinamidchlorid zunächst aus D,L-Carnitinnitrilchlorid gezielt hergestellt werden muß. Gegenüber einer Racematspaltung auf der Stufe des D,L-Carnitinnitrilchlorids, das direkt zu Carnitin verseift werden kann, ist also eine zusätzliche Stufe erforderlich.

Es sind auch Racematspaltungen auf der Carnitin-Vorstufe D,L-3-Chlor-2-hydroxypropyltrimethylammoniumchlorid bekannt, welche aber in technischem Maßstab nicht vollständig befriedigen - vgl` EP-A 0 157 315, EP-A 0 312 726.

Gemeinsame Merkmale einer Racematspaltung von D,L-Carnitinnitrilsalzen, wie beispielsweise D,L-Carnitinnitrilchlorid, dessen Herstellung im allgemeinen durch Cyanidierung von D,L-3-Chlor-2-hydroxypropyltrimethylammoniumchlorid oder D,L-Epoxypropyltrimethylammoniumchlorid erfolgt, sind die Überführung des Chlorids in das Hydroxid, Umsetzung mit einer optisch aktiven Säure, Trennung der diastereomeren Salze und Spaltung derselben mit einer starken Säure, wobei das optisch aktive Carnitinnitrilsalz gewonnen und die eingesetzte optisch aktive Säure zurückgewonnen werden.

Als optisch aktive Säure für die Racematspaltung von Carnitinnitrilchlorid wurden z. B. D-Weinsäure und D-Campher-10-sulfonsäure vorgeschlagen, welche aber wegen der geringen Löslichkeitsunterschiede ein häufiges Umkristallisieren der diastereomeren Salze erforderlich machten. Eine Verbesserung brachte zwar die kombinierte Verwendung von D-Campher-10-sulfonsäure und Dibenzoyl-L-weinsäure (E. Strack et al., Z. physiol. Chem 318, 129 (1960)), jedoch verursachen zwei Spaltreagenzien hohe Kosten und machen das Verfahren für eine industrielle Anwendung ungeeignet. Bei alleiniger Verwendung von Dibenzoyl-L-weinsäure ist der Löslichkeitsunterschied zwischen den beiden Diastereomeren gering, was wiederum die Ausbeute beeinträchtigt.

Zur Racematspaltung von D,L-Carnitinnitrilsalzen wurde auch die Verwendung von optisch aktiver N-Acetyl-glutaminsäure als Spaltsäure bekannt (JP-B 43-8248, NL-A 6614321). Bei dieser Racematspaltung ist nicht die natürlich vorkommende N-Acetyl-L-glutaminsäure, sondern deren Antipode N-Acetyl-D-glutaminsäure, welche selbst nicht in ausreichender Menge verfügbar ist bzw. selbst mittels D-Carnitinnitrilchlorid hergestellt werden muß, erforderlich. Verwendet man aber N-Acetyl-L-glutaminsäure als Spaltreagens und trennt zunächst das schwer lösliche Salz aus D-Carnitinnitril und N-Acetyl-L-glutaminsäure von dem Diastereomerengemisch ab. erfordert die Gewinnung eines optisch reinen L-Carnitin-N-acetyl-L-glutaminats aus der Mutterlauge ein mehrfaches fraktionierendes Kristallisieren, wobei erhebliche Ausbeuteverluste hinzunehmen sind.

Um die Effizienz der vorgenannten Racematspaltung zu verbessern und die optische Reinheit des gewünschten L-Carnitinnitrilsalzes zu erhöhen, wird in der JP-A 62-286959 (1987) vorgeschlagen, zunächst das Gemisch der diastereomeren Salze einer fraktionierenden Kristallisation zu unterwerfen, anschließend das optisch aktive Anion des überwiegend L-Carnitinnitril enthaltenden rohen Diastereomeren gegen ein optisch nicht aktives Säureanion, insbesondere Perchlorat oder Oxalat auszutauschen und das resultierende Salz mittels einer weiteren fraktionierenden Kristallisation zu reinigen. Durch diese zweite fraktionierende Kristallisation wird aber der Verfahrensaufwand wesentlich erhöht. Zudem vermindert sich vor allem bei der Racematspaltung im Produktionsmaßstab die Ausbeute, und es entstehen Kosten für den Einsatz und die Rückgewinnung der auszuwählenden optisch nicht aktiven Säure.

In der JP 62-286959 wird unter den Spaltreagenzien neben zahlreichen anderen auch optisch aktives N-Acetyl-prolin genannt, jedoch kein Hinweis gegeben, ob diese Säure in der L- oder D-Form eingesetzt werden soll. Im Hinblick auf das Ausführungsbeispiel unter Verwendung von N-Acetyl-L-glutaminsäure, wo das L-Carnitinnitril-N-Acetyl-L-glutaminat schwerer kristallisiert als das (DL)-Salz und die vorgeschlagene zweite fraktionierende Kristallisation erforderlich macht, konnte der Fachmann unter Verwendung von aus natürlich vorkommenden Rohstoffen, nämlich L-Prolin, leicht erhältlichem N-Acetyl-L-prolin ein vergleichbares Verhalten erwarten.

Im Verfahren der DE-Patentanmeldung P 40 15 573.0 werden optisch aktive N-Acetyl-2,2,5,5-tetraalkylthiazolidin-4-carbonsäuren als Spaltreagens zur Racematspaltung von D,L-Carnitinnitrilchlorid verwendet. Diese Spaltreagenzien sind aber nur auf synthetischem Wege zugänglich und erfordern ihrerseits eine Racematspaltung.

Aufgabe der Erfindung ist, ein gegenüber demjenigen der JP-A 62-286959 verbessertes Verfahren zur Herstellung von L-Carnitin aufzuzeigen, das eine Racematspaltung eines D,L-Carnitinnitrilsalzes und Verseifung des erhaltenen L-Carnitinnitrilsalzes umfaßt. Zur Racematspaltung sollte ein in ausreichender Menge in optisch aktiver Form vorkommendes, gegebenenfalls in einfacher Weise chemisch modifiziertes Spaltreagens Verwendung finden. Bei der Racematspaltung sollte zudem das L-Carnitinnitril enthaltende Diastereomere schwerer löslich und in hoher optischer Reinheit und hoher Ausbeute erhalten werden und damit eine zweite fraktionierende Kristallisation, wie sie im Verfahren der JP-A 62-286959 erforderlich war, überflüssig machen.

Gefunden wurde ein Verfahren zur Herstellung von L-Carnitin durch Racematspaltung von D,L-Carnitinnitrilsalzen, wobei das D,L-Carnitinnitrilsalz zur Bildung der diastereomeren Salze nach Überführung in das Hydroxid mit optisch aktivem N-Acetylprolin als Spaltreagens umgesetzt, eines der beiden diastereomeren Salze durch fraktionierende Kristallisation abgetrennt und die überwiegend das L-Carnitinnitril-Salz enthaltende Fraktion zum Zwecke der Abspaltung des optisch aktiven N-Acetylprolins mit einer optisch nicht aktiven starken Säure behandelt und das Spaltreagens abgetrennt wird, und Verseifung des erhaltenen L-Carnitinnitrilsalzes zu L-Carnitin, das dadurch gekennzeichnet ist, daß man als Spaltreagens N-Acetyl-L-prolin verwendet, wobei das diastereomere (LL)-Salz bei der fraktionierenden Kristallisation in hoher optischer Reinheit kristallin erhalten und das nach der Abspaltung und Abtrennung des N-Acetyl-L-prolins erhaltene L-Carnitinnitrilsalz ohne weitere Kristallisation der Verseifung zugeführt wird.

Im erfindungsgemäßen Verfahren wird in an sich bekannter Weise ein Salz aus dem D,L-Carnitinnitrilkation und dem Anion einer optisch nicht aktiven Säure, herstellungsbedingt handelt es sich häufig um das D,L-Carnitinnitrilchlorid, in das Hydroxid, also D,L-3-Cyano-2-hydroxypropyltrimethylammoniumhydroxid, überführt. Hierfür eignen sich insbesondere stark basische anorganische oder organische Ionenaustauscher in der OH⁻-Form oder die Elektrodialyse. Das D,L-Carnitinnitrilhydroxid wird vorzugsweise in wäßriger Lösung, mit N-Acetyl-L-prolin umgesetzt, wobei die diastereomeren (DL)- und (LL)-Salze gebildet werden. Das Molverhältnis D,L-Carnitinnitrilhydroxid zu N-Acetyl-L-prolin beträgt vorzugsweise etwa 1 zu 1. Es ist aber auch möglich, Molverhältnisse zwischen 1 zu 0,5 und 1 zu 1 zu wählen, wenn zusätzlich eine optisch nicht aktive organische Carbonsäure, wie insbesondere Essigsäure, mitverwendet wird, wobei das Molverhältnis von D,L-Carnitinnitrilhydroxid zur Summe aus N-Acetyl-L-prolin und der organischen optisch nicht aktiven Carbonsäure wiederum etwa 1 zu 1 beträgt.

Nach der Bildung des diastereomeren Salzgemisches und Entwässerung des Gemischs durch Abdestillieren unter vermindertem Druck und/oder azeotrope Destillation mit einem geeigneten organischen Lösungsmittel werden die diastereomeren Salze durch fraktionierende Kristallisation unter Verwendung organischer Lösemittel voneinander getrennt.

Die diastereomeren Salze mit dem N-Acetyl-L-prolinanion weisen beträchtliche Löslichkeitsunterschiede auf und gestatten eine bequeme Trennung der Diastereomeren. Überraschenderweise ist das diastereomere (LL)-Salz (L-Carnitinnitril-N-Acetyl-L-prolinat) schwerer löslich als das (DL)-Salz und läßt sich daher in hoher optischer Reinheit und hoher Ausbeute als Kristallisat gewinnen. Nach Abtrennung des Kristallisats von der das diastereomere (DL)-Salz (D-Carnitinnitril-N-Acetyl-L-prolin) enthaltenden Mutterlauge wird dieses, sofern erwünscht, umkristallisiert.

Als Lösungsmittel für die fraktionierende Kristallisation der diastereomeren Salzpaare werden vorzugsweise (C₁₋C₆)-Alkohole, insbesondere (C₁-C₄)-Alkohole, (C₃-C₇)-Ketone, wie insbesondere Aceton und Methylisobutylketon (MIBK), cyclische Ether, wie z. B. Tetrahydrofuran und Dioxan, Alkylenglykolether mit 3 bis 7 C-Atomen, insbesondere Ethylenglykolmonomethylether, oder Gemische solcher Lösungsmittel verwendet. Eine besonders bevorzugte Arbeitsweise zur Diastereomerentrennung besteht darin, das wasserfreie Diastereomerengemisch in einem primären C₃- bis C₅-Alkohol, insbesondere n-Butanol, zu lösen und durch Zugabe eines Ketons, insbesondere Aceton, das schwerer lösliche (LL)-Salz auszufällen. Zur Kristallisation des (LL)-Salzes aus dem Diastereomerengemisch werden n-Butanol und Aceton im Volumenverhältnis von 1 zu 1 bis 1 zu 4, insbesondere 1 zu 2,5 bis 3,5 , verwendet. Zur Erhöhung der optischen Reinheit kann man das auskristallisierte und abgetrennte (LL)-Salz noch waschen, zweckmäßig mit Aceton oder Methylisobutylketon, und/oder, soweit erforderlich, umkristallisieren, wobei zweckmäßigerweise die gleichen Lösungsmittel zur Anwendung kommen wie bei der Diastereomerentrennung. Besonders bevorzugt werden zum Umkristallisieren n-Butanol und Aceton im Volumenverhältnis von 1 zu 1,5 bis 2,5 verwendet.

Zur Abspaltung des N-Acetyl-L-prolins aus dem diastereomeren (LL)-Salz und Rückgewinnung des Spaltreagens wird das (LL)-Salz in an sich bekannter Weise mit einer starken, optisch nicht aktiven Säure behandelt. Gemäß einer bevorzugten Ausführungsform wird eine wäßrige Lösung des (LL)-Salzes zunächst mit einem Kationenaustauscher in der H⁺-Form behandelt und dann das N-Acetyl-L-prolin mittels Wasser vom L-Carnitinnitril-beladenen Kationenaustauscher abgetrennt; schließlich wird mittels einer optisch nicht aktiven, starken Säure, insbesondere Mineralsäure, vorzugsweise Salzsäure, das L-Carnitinnitrilsalz dieser Säure eluiert. Das Eluat enthält das L-Carnitinnitrilsalz der optisch nicht aktiven Säure und kann ohne weitere Reinigung oder gar Kristallisation des L-Carnitinnitrilsalzes der an sich bekannten Verseifung zu L-Carnitin zugeführt werden. Bei dieser Ausführungsform läßt sich das Spaltreagens auch im Produktionsmaßstab fast quantitativ - über 96 % - zurückgewinnen.

Alternativ, allerdings weniger bevorzugt, kann das diastereomere (LL)-Salz direkt mit einer optisch aktiven starken Säure umgesetzt werden, und zwar im Molverhältnis 1 zu 1. Das N-Acetyl-L-prolin wird dann aus dem von Wasser befreiten Reaktionsgemisch mittels eines geeigneten organischen Lösungsmittels, wie z. B. Aceton, oder eines Lösungsmittelgemischs, wie etwa Aceton/Ethanol, extrahiert, wobei das L-Carnitinnitrilsalz der verwendeten optisch nicht aktiven Säure als Rückstand verbleibt und der Verseifung zugeführt wird.

Selbstverständlich werden in der Regel auch die das diastereomere (DL)-Salz enthaltende Mutterlauge aus der Diastereomerentrennung sowie Mutterlaugen aus der Umkristallisation zur Rückgewinnung des Spaltreagens in der vorbeschriebenen Weise behandelt.

Die Überführung der L-Carnitinnitrilsalze in L-Carnitin erfolgt in bekannter Weise durch Erhitzen mit Mineralsäure, insbesondere Salzsäure und Abtrennung des Anions der Mineralsäure vom gebildeten L-Carnitinsalz, vorzugsweise unter Verwendung von Anionenaustauschern.

Die Verwendung von N-Acetyl-L-prolin als Spaltreagens erlaubt die Abtrennung des (LL)-Salzes in kristalliner, optisch reiner Form und in hoher Ausbeute. Eine zweite, dem Stand der Technik entsprechende, fraktionierende Kristallisation erübrigt sich damit. Zudem läßt sich das Spaltreagens fast quantitativ, meist zwischen 97 und 99,5 %. zurückgewinnen. Ausgehend von (DL)-Carnitinnitrilchlorid ist L-Carnitin im erfindungsgemäßen Verfahren in über 70 %iger Ausbeute und hoher Enantiomerenreinheit zugänglich.

### Beispiel 1

### Racematspaltung von D,L-Carnitinnitrilchlorid mit L-N-Acetylprolin

a) Bildung der diastereomeren Salze und Abtrennung des (LL)-Salzes.
1 Mol einer ca. 6 %igen, wäßrigen D,L-Carnitinnitrilhydroxid-Lösung wurde durch Zusatz von 158,2 g (1,006 Mol) L-N-Acetylprolin (L-NAP, [α]_{D}²⁰ = -116 ^{o}C = 1, H₂O) auf pH 5,5 gestellt und die schwach gelb gefärbte Lösung im Vakuum bei 60 °C eingeengt. Der Rückstand (336 g gelbes Öl) wurde in 250 ml n-Butanol gelöst und zur vollständigen Entwässerung im Teilvakuum (Sumpftemperatur 70 °C) über einen Wasserabscheider andestilliert (ca. 30 ml wäßrige Phase). Man erhielt eine Lösung, die bei Siedetemperatur mit 750 ml Aceton versetzt wurde. Beim Erkaltenlassen auf Raumtemperatur begann die Lösung zu kristallisieren. Die Suspension wurde noch 2 h bei Raumtemperatur gerührt, der Niederschlag abgesaugt und mit Aceton gewaschen. Das acetonfeuchte Rohsalzpaar versetzte man anschließend zur Umkristallisation mit 200 ml n-BuOH und erhitzte unter Destillation von restlichem Aceton (ca. 35 ml). Bei ca. 100 °C ging der Feststoff in Lösung. Während die Lösung siedete, wurden langsam 400 ml Aceton zugesetzt, wobei ein Niederschlag ausfiel. Nach Erkaltenlassen auf Raumtemperatur wurde noch 2 h bei dieser Temperatur gerührt. Anschließend saugte man den Feststoff ab, wusch mit Aceton und trocknete bei 70 °C im Vakuum:
117,0 g (0,391 Mol; 78.2 % d. Th.) farbloses (LL)-Salz.

| Analyse ber. für C₁₄H₂₅N₃O₄ (299,37): | | | |
|---|---|---|---|
| | C 56,17 | H 8,42 | N 14,04 |
| gef. | C 56,01 | H 8,55 | N 14,23 |

[α]_{D}²⁰ = -69,4 ° (c=1, Wasser)
b) Spaltung des diastereomeren (LL)-Salzes
104.8 g (0.35 Mol) (LL)-Salz wurden in ca. 900 ml Wasser gelöst und über eine Glassäule, die mit 0,5 l Ionenaustauscherharz Lewatit SP 112 / H⁺-Form gefüllt war, gepumpt (Verweilzeit: 2 h). Der pH-Wert im Eluat fiel auf pH 1,8 ab und stieg im Verlauf der Eluation mit Wasser langsam wieder an. Die Eluate bis pH 3,3 wurden nach titrimetrischer Gehaltsbestimmung (0.1 n NaOH) vereinigt, im Vakuum eingeengt und der verbliebene Rückstand im Vakuum bei 75 °C getrocknet:
54,8 g (0,349 Mol; 99,6 %) schwach gefärbtes L-N-Acetylprolin. [α]_{D}²⁰ = -115,8 ° (c=1, Wasser)
Die Ionenaustauschersäule wurde nach Spülen mit Wasser auf pH 4-5 mit 10 %iger Salzsäure eluiert. Man pumpte solange Salzsäure durch die Säule, bis im Eluat mit Reinecke-Salz kein Carnitinnitrilchlorid mehr nachzuweisen war.
Durch das Eluieren mit Salzsäure ist der Ionenaustauscher nach Spülen mit Wasser bereits für die nächste Salzpaarspaltung wieder regeneriert.
c) Spaltung des diastereomeren (DL)-Salzes
Die vereinigten Mutterlaugen der (LL)-Salz-Fällung sowie der -Umkristallisation wurden zur Entfernung der organischen Lösungsmittel zunächst bei Normaldruck bis 70 °c (Aceton) und anschließend im Teilvakuum destilliert (n-BuOH). Den öligen Rückstand löste man in 1500 ml Wasser und strippte zur Entfernung des Rest-Butanols im Vakuum an. Die leicht gefärbte Lösung wurde zur Salzpaarspaltung über den zuvor beschriebenen stark sauren Ionenaustauscher gepumpt. Das Eluat (bis pH 3,3 - Gehaltskontrolle durch Titration) wurde einrotiert und im Vakuum bei 70 °C getrocknet:
93,0 g (0,592 Mol; 96,2 %) schwach gefärbtes L-N-Acetylprolin. [α]_{D}²⁰ = -115,8 ° (c=1, Wasser)
Die Gesamtrückgewinnung an L-N-Acetylprolin betrug damit 97,4 %.
Im Recycle-Betrieb werden die wäßrigen L-NAP-Eluate vereinigt. aufkonzentriert und als wäßrige ca. 13 %ige Lösung (Löslichkeit von L-NAP in Wasser bei Raumtemperatur: 16 %) wieder in den Spaltprozeß zurückgeführt.
d) Verseifung von L-Carnitinnitrilchlorid und Isolierung von L-Carnitin
Die salzsauren L-Carnitinnitrilchlorid-Eluate aus der (LL)-Salz-Spaltung wurden im Vakuum zur Trockene einrotiert, der ölig-kristalline Rückstand mit 120 g 37 %iger Salzsäure (1,23 Mol) versetzt und unter Standardbedingungen verseift. Nach Abkühlen auf 5 °C saugte man das ausgefallene Ammoniumchlorid ab und wusch mit kalter konz. HCl nach. Das Filtrat wurde im Vakuum bis zur Trockene einrotiert. Der ölig-kristalline Rückstand wurde in 300 ml Wasser gelöst und über eine mit 0,5 l Amberlite IRA 410/OH⁻-Form gefüllte Glassäule gepumpt. Die klare Lösung wurde anschließend eingeengt, der Rückstand in 125 ml n-BuOH gelöst und zur vollständigen Entwässerung am Wasserabscheider im Teilvakuum erhitzt. Dabei fiel bereits ein kristalliner Niederschlag aus. Bei Siedetemperatur wurde anschließend mit 125 ml Aceton versetzt und weitere 30 min unter Rückfluß gerührt. Nach Erkaltenlassen auf 20 °C ließ man 1 h nachrühren, saugte den Niederschlag ab, wusch mit Aceton nach und trocknete bei 75 °C im Vakuum:
51,0 g (0,316 Mol) farbloses L-Carnitin (90,2 % bezogen auf (LL)-Salz). [α]_{D}²⁰ = -31,7 ° (c=1, Wasser) HPLC: >99 %

### Beispiel 2

### Bildung der diastereomeren Salze von D,L-Carnitinnitrilhydroxid mit L-N-Acetylprolin und Essigsäure

0,5 Mol einer ca. 6 %igen D,L-Carnitinnitrilhydroxid-Lösung wurden mit 47,15 g (0,3 Mol) L-N-Acetylprolin und 12 g (0,2 Mol) Essigsäure versetzt. Man erhielt eine schwach gefärbte Lösung mit pH 6. Nach Einengen der Salzpaar-Lösung im Vakuum erhielt man 155.3 g eines Öls, das mit 125 ml n-Butanols versetzt und zur vollständigen Entwässerung im Teilvakuum über einen Wasserabscheider destilliert wurde (25 ml wäßrige Phase). Zu dem Rückstand gab man bei Siedetemperatur 375 ml Aceton. Beim Erkaltenlassen der Lösung fiel ein kristalliner Niederschlag aus, der nach Absaugen und Waschen mit Aceton in 100 ml n-BuOH erhitzt wurde. Während des Abdestillierens von Aceton ging bei ca. 100 °C der Niederschlag in Lösung. Bei Siedetemperatur wurde anschließend 200 ml Aceton zugesetzt, wobei Kristallbildung einsetzte. Nach Erkaltenlassen auf 20 °C rührte man noch 2 h nach, saugte den Niederschlag ab, wusch mit Aceton nach und trocknete bei 75 °C im Vakuum:
51,5 g (0,172 Mol; 68,8 % d. Th.) farbloses (LL)-Salz.

Die Untersuchung des diastereomeren Salzes mit dem Chira-Monitor (HPLC) ergab für den L-Carnitinnitril-Anteil im Salz 96,9 % ee.

15 g (50 mmol) (LL)-Salz wurden in Wasser gelöst und mit Salzsäure auf pH 2 gestellt. Anschließend rotierte man die Lösung zur Trockene ein und nahm den Rückstand in Ethanol auf. Dann wurde erneut zur Trockene einrotiert und der Rückstand mit 50 ml Aceton verrührt. Das unlösliche L-Carnitinnitrilchlorid wurde abgesaugt und mit Aceton gewaschen. Nach Trocknen erhielt man 8,5 g (47,6 mmol; 95,2 % bezogen auf das LL-Salz) L-Carnitinnitrilchlorid mit einem Drehwert von [α]_{D}²⁰ = -26,4 ° (c=1, Wasser).

### Beispiel 3

Gemäß Beispiel 1, jedoch unter Einsatz von 120 Mol D,L-Carnitinnitrilchlorid, wurde die Racematspaltung durchgeführt. Zur Abtrennung des rohen (LL)-Salzes wurden pro Mol der diastereomeren Salze 250 ml n-Butanol zum Lösen und 750 ml Aceton zum Ausfällen verwendet. Zum Umkristallisieren wurde mit 200 ml n-Butanol pro Mol Salz gelöst und mit 400 ml Aceton ausgefällt. Ausbeute an reinem (LL)-Salz: 52,3 Mol; 87,2 % der Theorie; Enantiomerenreinheit des L-Carnitinnitrilchlorids, bestimmt durch Vermessen des Salzes am Chira-Monitor (HPLC, externer Standard): 96,8 % ee. Bei der Spaltung des (LL)-Salzes gemäß Beispiel 1b wurden 96,9 % des eingesetzten N-Acetyl-L-prolins zurückgewonnen. Die salzsauren L-Carnitinnitrilchlorid-Eluate wurden gemäß Beispiel 1d in L-Carnitin überführt.

## Patentansprüche

1. Verfahren zur Herstellung von L-Carnitin durch Racematspaltung von D,L-Carnitinnitrilsalzen, wobei das D,L-Carnitinnitrilsalz zur Bildung der diastereomeren Salze nach Überführung in das Hydroxid mit optisch aktivem N-Acetylprolin als Spaltreagens umgesetzt, eines der beiden diastereomeren Salze durch fraktionierende Kristallisation abgetrennt und die überwiegend das L-Carnitinnitril-Salz enthaltende Fraktion zum Zwecke der Abspaltung des optisch aktiven N-Acetylprolins mit einer optisch nicht aktiven starken Säure behandelt und das Spaltreagens abgetrennt wird, und Verseifung des erhaltenen L-Carnitinnitrilsalzes zu L-Carnitin,
dadurch gekennzeichnet,
daß man als Spaltreagens N-Acetyl-L-prolin verwendet, wobei das diastereomere (LL)-Salz bei der fraktionierenden Kristallisation in hoher optischer Reinheit kristallin erhalten und das nach der Abspaltung und Abtrennung des N-Acetyl-L-prolins erhaltene L-Carnitinnitrilsalz ohne weitere Kristallisation der Verseifung zugeführt wird.

2. Verfahren nach Anspruch 1.
dadurch gekennzeichnet,
daß man das D,L-Carnitinnitrilsalz in wäßriger Lösung durch Behandlung mit einem stark basischen Ionenaustauscher in das D,L-Carnitinnitrilhydroxid überführt.

3. Verfahren nach Anspruch 1 oder 2,
dadurch gekennzeichnet,
daß man aus dem Gemisch der diastereomeren Salze das (LL)-Salz auskristallisiert, indem zu einer wasserfreien Lösung der diastereomeren Salze in einem (C₁-C₆)-Alkohol oder Alkoholgemisch, insbesondere in n-Butanol, ein (C₃-C₇)-Keton oder Ketongemisch, insbesondere Aceton, zugegeben wird.

4. Verfahren nach einem der Ansprüche 1 bis 3,
dadurch gekennzeichnet,
daß man das in kristalliner Form abgetrennte diastereomere (LL)-Salz, falls erforderlich nach Umkristallisation desselben, zur Abspaltung und Rückgewinnung des N-Acetyl-L-prolins mit einem stark sauren Ionenaustauscher behandelt und durch Eluation mittels einer starken, optisch nicht aktiven Säure das L-Carnitinnitrilsalz dieser Säure in Form einer wäßrigen Lösung gewinnt.

5. Verfahren nach Anspruch 3,
dadurch gekennzeichnet,
daß man zur Kristallisation des (LL)-Salzes n-Butanol und Aceton im Volumenverhältnis von 1 zu 1 bis 1 zu 4, insbesondere 1 zu 2,5 bis 3,5 , verwendet.

## Claims

1. Method for the preparation of L-carnitine by separation of a racemic mixture of D,L-carnitine nitrile salts, wherein the D,L-carnitine nitrile salt, after conversion to the hydroxide, is reacted with optically active N-acetylproline as splitting reagent in order to form the diastereoisomeric salts, one of the two diastereoisomeric salts is separated off by fractional crystallisation and the fraction predominantly containing the L-carnitine nitrile salt is treated with an optically inactive strong acid for the purpose of splitting off the optically active N-acetylproline, and the splitting reagent is separated off, and saponification to L-carnitine of the L-carnitine nitrile salt obtained,
characterised in that
the splitting reagent used is N-acetyl-L-proline, wherein the diastereoisomeric (LL)-salt is obtained crystalline in high optical purity during the fractional crystallisation and the L-carnitine nitrile salt obtained after the splitting off and separation of the N-acetyl-L-proline is removed for saponification without further crystallisation.

2. Method according to claim 1,
characterised in that
the D,L-carnitine nitrile salt in aqueous solution is converted to the D,L-carnitine nitrile hydroxide by treatment with a strongly basic ion exchanger.

3. Method according to claim 1 or 2,
characterised in that
the (LL)-salt is crystallised out from the mixture of diastereoisomeric salts by adding a C₃-C₇ ketone or ketone mixture, particularly acetone, to an anhydrous solution of the diastereoisomeric salts in a C₁-C₆ alcohol or alcohol mixture, particularly in n-butanol.

4. Method according to one of claims 1 to 3,
characterised in that
the diastereoisomeric (LL)-salt separated in crystalline form, if necessary after recrystallisation of the same, is treated with a strongly acidic ion exchanger for the splitting off and recovery of the N-acetyl-L-proline and, through elution by means of a strong, optically inactive acid, the L-carnitine nitrile salt of the said acid is isolated in the form of an aqueous solution.

5. Method according to claim 3,
characterised in that
n-butanol and acetone in the volume ratio of from 1 to 1 up to 1 to 4, particularly from 1 to 2.5 up to 3.5, are used for the crystallisation of the (LL)-salt.

## Revendications

1. Procédé d'obtention de L-carnitine par dédoublement des racémates de sels de D,L-carnitine-nitrile, dans lequel le sel de D,L-carnitine-nitrile est mis à réagir en vue de la formation des sels diastéréo-isomères après conversion en hydroxyde, avec de la N-acétylproline optiquement active comme réactif de dédoublement, dans lequel on sépare un des deux sels diastéréo-isomères par cristallisation fractionnante et la fraction qui contient d'une façon prédominante le sel de L-carnitine-nitrile est traitée aux fins de la séparation de la N-acétylproline optiquement active, avec un acide fort optiquement inactif optiquement et le réactif de dédoublement est séparé, puis saponification du sel de L-carnitine-nitrile obtenu en L-carnitine, caractérisé en ce que l'on utilise comme réactif de dédoublement la N-acétyl L-proline, avec laquelle le sel diastéréo-isomère (LL) est obtenu lors de la cristallisation fractionnante sous forme cristalline, avec une pureté optique élevée et que l'on apporte le sel de carnitine-nitrile obtenu après le dédoublement et la séparation de la N-acétyl L-proline, sans autre cristallisation, à la saponification.

2. Procédé selon la revendication 1, caractérisé en ce que l'on convertit le sel de D,L carnitine-nitrile en solution aqueuse par traitement avec un échangeur d'ions fortement basique en hydroxyde de D,L-carnitine nitrile.

3. Procédé selon la revendication 1 ou la revendication 2, caractérisé en ce que l'on sépare par cristallisation du mélange de sels diastéréo-isomères, le sel (LL), en ajoutant à une solution anhydre des sels diastéréo-isomères dans un alcool en C₁ à C₆ ou un mélange d'alcools, en particulier dans du n-butanol, une cétone en C₃ à C₆ ou un mélange de cétones, en particulier de l'acétone.

4. Procédé selon l'une des revendications 1 à 3, caractérisé en ce que l'on traite le sel diastéréo-isomère (LL) séparé sous forme cristalline au cas où c'est nécessaire après recristallisation de celui-ci, en vue du dédoublement et de la récupération de la N-acétyl-L-proline, avec un échangeur d'ions fortement acide et que l'on obtient par élution à l'aide d'un acide fort, non actif optiquement, le sel de L-carnitine-nitrile de cet acide sous forme d'une solution aqueuse.

5. Procédé selon la revendication 3, caractérisé en ce que l'on utilise pour la cristallisation du sel (LL), du n-butanol et de l'acétone en rapport volumique allant de 1 pour 1 à 1 pour 4, en particulier de 1 pour 2,5 à 3,5.
